(19) Europäisches Patentamt
European Patent Office
Office européen des brevets

(11) Publication number: **0 126 780**
**A1**

(12) # EUROPEAN PATENT APPLICATION

(21) Application number: 83103507.6

(22) Date of filing: 12.04.83

(51) Int. Cl.³: C 07 D 487/04
A 61 K 31/40
//C07D205/08, (C07D487/04, 209/00, 205/00)

(43) Date of publication of application:
05.12.84 Bulletin 84/49

(84) Designated Contracting States:
AT BE CH DE FR GB IT LI NL SE

(71) Applicant: SCHERING CORPORATION
2000 Galloping Hill Road
Kenilworth, New Jersey 07033(US)

(72) Inventor: Afonso, Adriano
10 Woodmere Road
West Caldwell New Jersey 07006(US)

(74) Representative: Antony, Fritz, Dr. et al,
c/o Scherico Ltd. P.O. Box 601 Töpferstrasse 5
CH-6002 Lucerne(CH)

(54) **Carbapenem compound.**

(57) (5R,6S,8R,2'S)-2[(2'-amino-2'-carboxyethyl)thio]- 6-(1-hydroxyethyl)-1-carbapen-2-em-3-carboxylic acid sodium salt, methods for producing it, a pharmaceutical composition containing it, and its use in the treatment of bacterial infections.

EP 0 126 780 A1

Croydon Printing Company Ltd.

A CARBAPENEM COMPOUND

The present invention relates to the compound (5R,6S,8R,2'S)-2[(2'-amino-2'carboxyethyl)thio]-6-(1-hydroxyethyl)-1-carbapen- -2-em -3-carboxylic acid sodium salt, and to pharmaceutical compositions containing it.

Specifically the compound of the present invention is the mono sodium salt, and it will be appreciated that there will be a greater tendency for salt formation to occur at the 3-carboxyl group rather than the other carboxyl group. Compared with the free acid the sodium salt is more stable and has greater solubility for instance in pharmaceutical carriers, and is more readily absorbed in animal (e.g. human) bodies.

Further the present compound has the stereochemistry 5R,6S,8R,2'S. Compared with similar compounds which have the R configuration at the 2' position the present compound is surprisingly found to have a broader spectrum and greater potency.

Compounds similar to the present compound are disclosed in European Patent Publication No. 17992.

The compound of this invention possesses antibacterial activity of the gram positive and gram negative type. Thus when tested in standardized microbiological assays, it is active against such gram positive organisms as Staphylococcus epidermis and Bacillus subtilis, and such gram negative organisms as E. coli, Pseudomonas and Salmonella at test levels of 0.1 to 100 mg/ml. Additionally it shows activity against such organisms in the presence of penicillinase indicating a resistance to this enzyme, and is an inhibitor of β-lactamase.

Thus the present invention includes within its scope pharmaceutical compositions comprising the compound of the invention together with a pharmaceutically acceptable carrier or coating.

The dosage administered of the compound of the invention is dependant upon the age and weight of the animal species being treated, the mode of administration, and the type and severity of bacterial infection being prevented or reduced. Typically, the dosage administered per day will be in the range of 100 - 5000 mg, with 500 - 1000 mg being preferred.

For oral administration, the compound of the invention may be formulated in the form of tablets, capsules, elixirs or the like. Likewise it may be admixed with animal feed. It may also be applied topically, in the form of ointments, both hydrophilic and hydrophobic, in the form of lotions which may be aqueous, non-aqueous or of the emulsion type, or in the form of creams.

The compound of this invention may be prepared by a process characterized in that

A: (5R,6S,8R,2'S) -2[(2'-amino-2'-carboxyethyl)thio] -6-(1-hydroxyethyl)-1-carbapen-2-em-3-carboxylic acid is transformed into its sodium salt;

B: a compound of general formula I

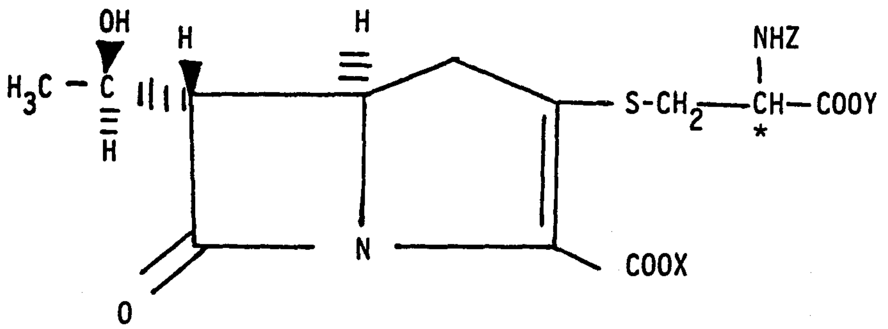

(I),

wherein * indicates a chiral centre of S configuration, Z is hydrogen or an amino protecting group and one of X and Y is a carboxy protecting group and the other is hydrogen or a carboxy protecting group, is subjected to deprotection by catalytic dehydrogenation in the presence of sodium cation; or

C: a compound of formula I as defined above in which Z is hydrogen, is subjected to catalytic deprotection in the presence of sodium cation.

The free acid used as starting material in process A can be obtained by deprotecting a compound of formula I by any convenient known method.

For instance, when in a compound of formula I X and/or Y are ester groups such as benzyl, p-nitrobenzyl and benzhydryl these can be removed by e.g. hydrogenation in the presence of a transition metal catalyst. The pressure of hydrogen used may be low, medium or high but generally slightly super-atmospheric. Palladium or charcoal is a suitable transition metal catalyst. The reaction is generally carried out in an inert solvent e.g. methylene chloride or aqueous dioxan. If no base is present the acid (X and Y=H) in formula I is prepared, which can then be partly neutralized to yield the desired sodium salt using e.g. Na OH or $NaHCO_3$. If the hydrogenation is carried out in the presence of an appropriate amount of such a base, then the sodium salt is produced directly (process B).

The conventional amino protecting groups Z are e.g. benzyloxycarbonyl, p-nitrobenzyloxycarbonyl and benzhydryloxy carbonyl, which can likewise be removed by hydrogenation.

Alternatively the free acid of process A can be obtained by removing the protecting groups X, Y and Z wherein X and Y are allylic groups and Z is allylic oxycarbonyl by the method described in European Patent Application Publication No. 13663. This method preferably uses a suitable aprotic solvent such as tetrahydrofuran, diethylether or methylene chloride with e.g. 2-ethylhexanoic acid in the presence of a mixture of a palladium compound and triphenylphosphine as a catalyst.

If in the immediately preceding procedure Z is hydrogen rather than a protecting group and the reaction is carried out in the presence of e.g. sodium 2-ethylhexanoate, the desired sodium salt of the invention can be obtained directly (process C).

Another method of obtaining the free acid is by removing allyl and allyloxycarbonyl protecting groups using the procedure of Tsuji described in Tetrahedron letters 7 613 (1979) which utilizes an amine salt of formic acid and a mixture of a palladium compound and triphenyl phosphine as catalyst to yield the free acid which can then be treated by a base as aforesaid to give the desired sodium salt. Preferably the base used is sodium 2-ethyl hexanoate in a solvent such as ethyl acetate.

A compound of formula I is preparable by reacting a compound of formula II

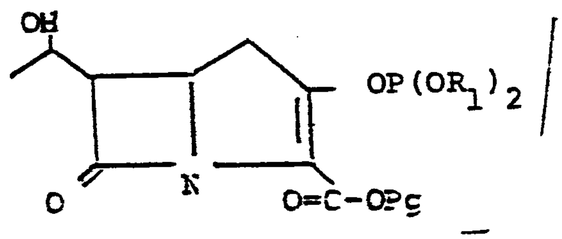

II

in which the hydroxy group at position 8 is optionally protected, $R_1$ is alkyl or aryl, and Pg is a carboxy protecting group, with a compound of formula III

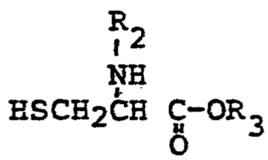

III

in which $R_2$ is an amino protecting group and $R_3$ is a carboxy protecting group,

if desired separating a mixture of stereoisomers before or after optionally removing the protecting groups.

It is possible to prepare a compound of formula I by stereospecific synthesis starting from compounds of formulae II and III of appropriate stereochemistry. Alternatively if stereoisomeric mixtures of the compound of formula II and/or formula III are used a mixture of stereoisomers of formula I is obtained.

The separation of steroisomers where necessary or desired, may be carried out by conventional means such as chromatography or optically active salt formation.

The separation of racemic mixtures e.g. a mixture of a 5R,6S,8R,2'S isomer with its enantiomer 5S,6R,8S,2'R can be carried out by resolution by fractional crystallization of optically active salt forms e.g. the salts derived from optically active amino acids, (-) - brucine or (+) - and (-) ephodrine.

The starting materials of formula II are preparable from the known compound 4-allylazetidin-2-one, via the following reaction sequence

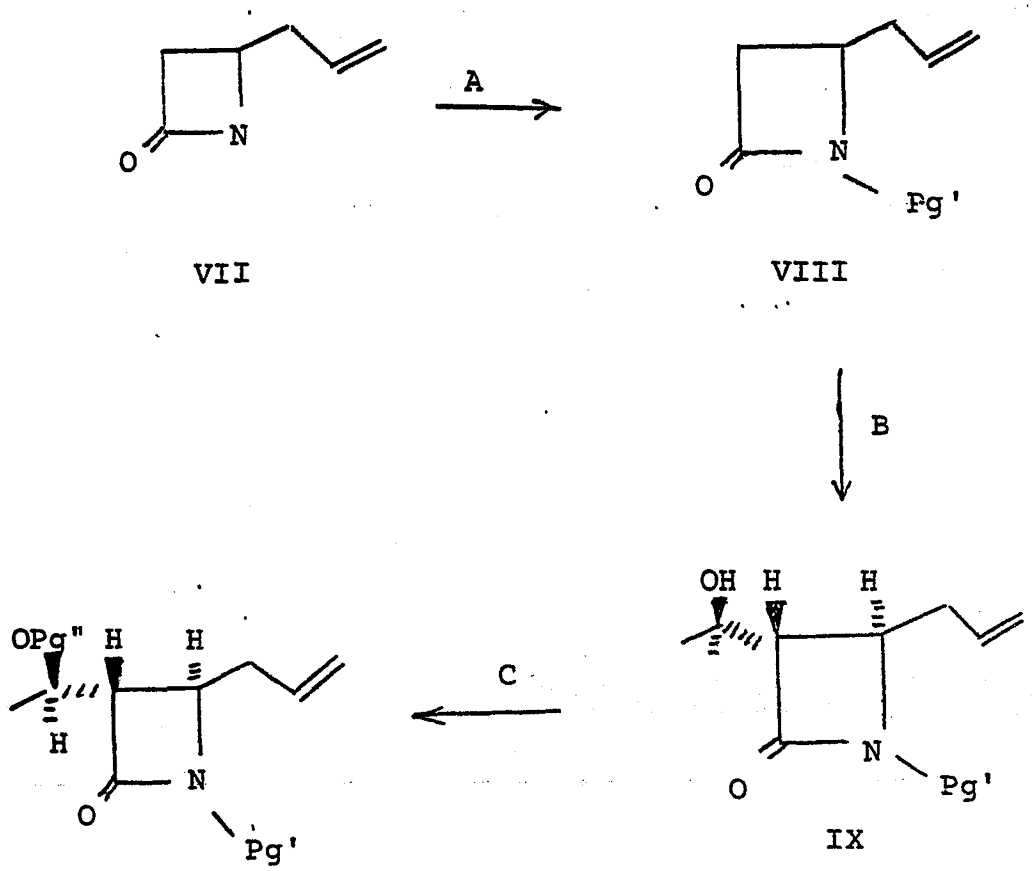


VII

VIII

IX

X

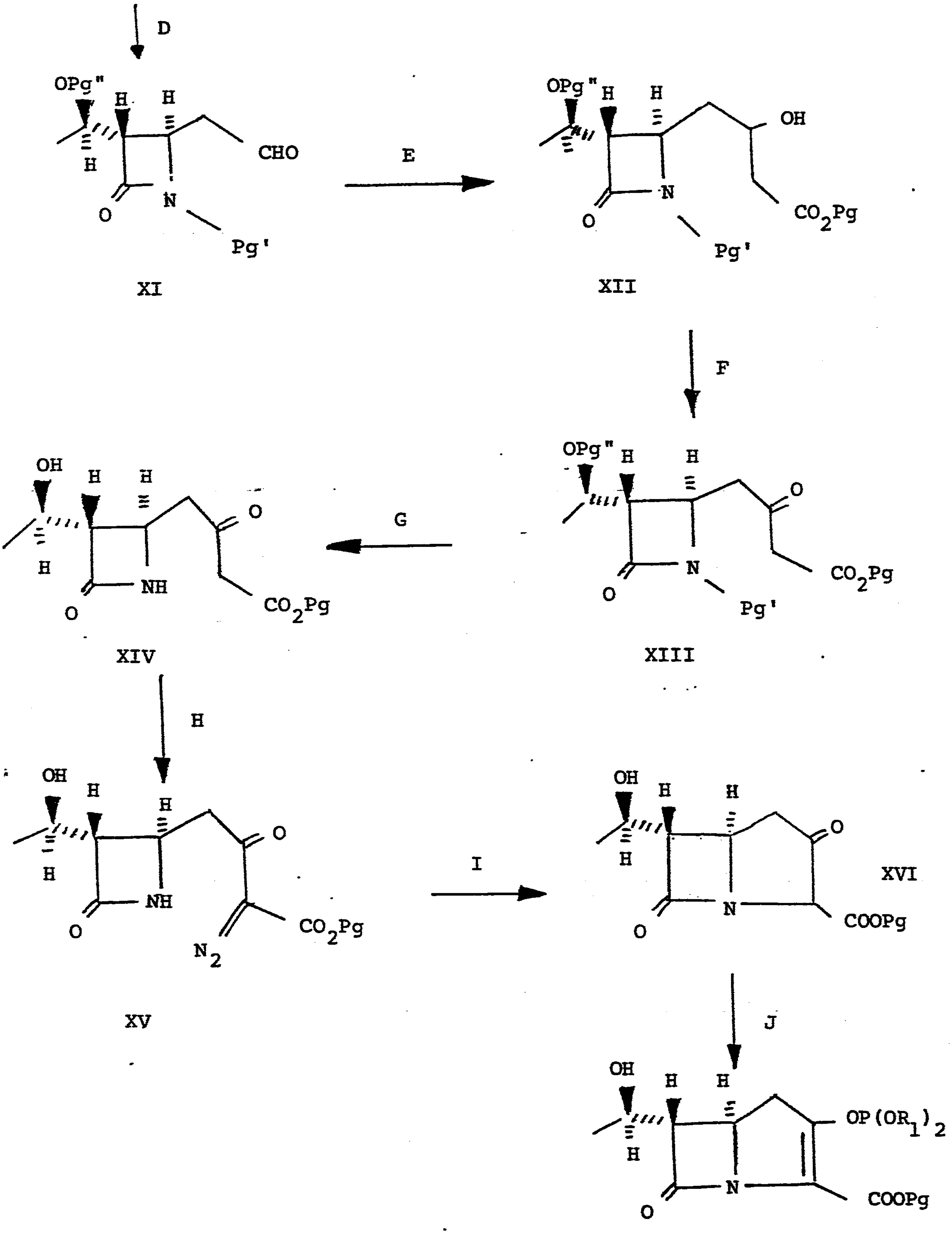


XI

XII

XIII

XIV

XV

XVI

I

In step A of the above scheme the 4-allylazetidinone -2-one of formula VII is reacted with a trialkylsilyl-chloride of the formula Pg'-Cl wherein Pg' is the trialkylsilyl group, in the presence of an acid acceptor such as triethylamine so as to provide the N-protected compound of formula VIII. Preferably, the reaction is conducted in an organic solvent, particularly a halogenated hydrocarbon solvent such as chloroform or methylene chloride.

In step B the compound of formula VIII is converted to the $R_4$ substituted compound IX. The $R_4$ substituent is most conveniently added by treatment with lithium diisopropylamine and the appropriate aldehyde in an anhydrous aprotic solvent, especially tetrahydrofuran.

The preparation of compounds of formula IX from a compound of formula VII is also described in European Patent Application Publication No. 8497.

In step C, the various isomers of the compounds of formula IX are separated, by conventional means i.e. chromatography or optically active salt formation. Additionally the hydroxy group in the 5-position is protected prior to step D using a conventional hydroxy protecting group. Suitable groups are trichloroethoxycarbonyl and t- butyldimethylsilyl, but others such as p-nitrobenzyloxycarbonyl or allyloxycarbonyl may be used.

Step D converts the 4-allyl substituent of a compound of formula X to a 4 ethanal substituent in the compound of formula XI, typically using a conventional ozonolysis procedure.

In step E the aldehyde of formula XI is treated with a suitable acetate ester anion in an aprotic solvent such as tetrahydrofuran or ethyl ether. The product which results is the beta-hydroxyester of formula XII. A preferred acetate ester anion for use in this reaction is the lithium salt of allylacetate produced by adding lithium diisopropylamide to allyl acetate at low temperature. Pg in the reaction sequence is a carboxy protecting group such as allyl or p-nitrobenzyl.

Conversion of compound XII to compound XIII in step F is accomplished by treatment of the compound XII with an oxidising agent such as chromium trioxide. Preferably a halogenated hydrocarbon such as chloroform or methylene chloride is utilized as solvent.

In step G the hydroxy protecting group Pg" is removed and also nitrogen protecting group Pg'. The method of removal obviously depends upon the particular protecting groups used. Trichloroethoxy carbonyl groups come off using zinc/acetic acid while N-t-butyldimethylsilyl groups are removeable with hydrochloric acid or amberlite 401-S(F) resin.

Step H involves diazonation of the compound of formula XIV to afford compound XV. Typically this is accomplished using p-carboxybenzenesulfonylazide in the presence of an acid acceptor. Preferred temperatures range from about -5 to about $5^{O}$ C. Preferred solvents are halogenated hydrocarbons such as methylene chloride and chloroform.

Step I. involves treatment of compound XV with rhodium acetate at a temperature of $40-100^{O}$ C in a suitable organic solvent such as benzene or toluene to afford the

intermediate of the formula XVI, which is thereafter reacted in step J with a dialkyl or diarylchlorophosphate in the presence of an organic base to afford the compound II.

Suitable organic bases are those such as pyridine, triethylamine and diisopropylethylamine.

Suitable dialkyl and diarylchlorophosphates are those such as di-n-butyl and diphenylchlorophosphate.

In the process of the invention the compound II is preferably not isolated but directly reacted with the compound III.

In the following Example "NMR" denotes nuclear magnetic resonance spectra; "MS" denotes mass spectra; "UV" denotes ultra violet spectra and "IR" denotes infrared spectra. Chromatography is performed on silica gel unless denoted otherwise.

PREPARATION A

A. 1-(t-butyldimethyl)silyl-4-allylazetidin-2-one: t-Butyldimethylsilylchloride (7.25 g) is added to a solution of 4-allylazetidin-2-one (5 g) in methylene chloride (50 ml) and triethylamine (5 ml) at 0°C. The mixture is stirred for 2 hours, washed successively with water and brine, dried and evaporated to afford the title product as a colorless oil having IR: 5.70 ;NMR: ($CDCl_3$) 0.2(3H), 1.0(6H).

B. 1-(t-butyldimethyl)silyl-3(S)-[1'(R)-hydroxyethyl]-4(R)-allylazetidin-2-one: A solution of 1-(t-butyldimethyl)silyl-4-allylazetidin-2-one 22.4 g) in tetrahydrofuran (200 ml) at -76°C is added during 10 minutes to a cold solution (-76°C) of lithium diisopropylamide prepared from 2.5 M butyllithinum (44 ml) and diisopropylamine (15.4 ml) in dry tetrahydrofuran (380 ml). After stirring for 10 minutes, freshly distilled acetaldehyde (12 ml) is added to the reaction mixture. The reaction is quenched after 60 seconds by adding

saturated solution of ammonium chloride (150 mg), allowed to warm to 0°C and then extracted three times with 250 ml portions of ethyl acetate. The extracts are combined, washed with brine, dried and the solvents removed. The resulting oil is separated by preparative HPLC on 2 Prep. 500 silica gel columns using 30:60 ethyl acetate-hexane as solvent to afford the desired compound as an oil, having IR: 5.70 ; NMR: ($CDCl_3$) 0.2(3H), 1.0(6H), 1.28(d,3H,J=6 cps), 2.85 (dd,1H,J=6;3 cps); MS: 270 (M+ +1).

The 1'(S) isomer (3.5 g) and the 4(S) isomer (3.8 g) which are formed in the reaction, are separable from the desired compound.

C. <u>1-(t-Butyldimethyl)silyl-3(S)-[1'(R)-2,2,2-trichloroethoxycarbonyloxyethyl]-4(R)-allylazetidin-2-one</u> :

A solution of 1-(t-butyldimethyl)silyl-3(S)-[1'(R)-hydroxyethyl]-allylazetidin-2-one (8 g) in methylene chloride (80 ml) is cooled to 0°C and treated with pyridine (3.4 ml) followed by dropwise addition of trichloroethoxychloroformate (5.5 ml). The mixture is stirred for 1 hour, washed three times with 20 ml portions of water and then brine, dried and evaporated to afford the title compound as an oil having IR: 5.75 ; NMR: ($CDCl_3$) 4.72(s, 2H), 3.0 (dd, 1H, J=7; 3 cps); MS: 386, 388 (M+ -57).

D. 1-(t-butyldimethyl)silyl-3(S)-[1'(R)-2,2,2-trichloroethoxycarbonyloxyethyl]-4(R)-(2''-ethanal)azetidin-2-one:

Ozonized oxygen is bubbled through a solution of 1-(t-butyldimethyl)silyl-3(S)-[1'(R)-2,2,2-trichloroethoxycarbonyloxyethyl]-4(R)-allylazetidin-2-one (14 g) in methylene chloride (80 ml) at -76°C until layer chromatography (silica gel plates, 40:60 ethyl acetate:hexane) indicates disappearance of starting material. Excess ozone is swept off the solution with a stream of nitrogen followed by addition of dimethylsulfide (8 ml). The solution is allowed to stand at room temperature overnight and then is evaporated to afford the title compound as an oil having NMR: ($CDCl_3$) 3.06(dd, 1H, J=7.3 cps); 7.20 (S, 1H): MS: 388, 390 (M+ -57).

E. 1-(t-butyldimethyl)silyl-3(S)-[1'(R)-2,2,2-trichloroethoxycarbonyloxyethyl]-4(R)-(allyl-3''-hydroxy-4''-butyrate)-azetidin-2-one:

Allyl acetate (1 ml) is added to a solution of lithium diisopropylamide (prepared by adding 2.8 ml of a 2.5M solution n-butyllithium to 1 ml diisopropylamide in 20 ml tetrahydrofuran) at -70°C followed after 10 minutes by a solution of 1-(t-butyldimethyl)silyl-3(S)-[1'(R)-2,2,2-trichloroethoxycarbonyloxyethyl]-4(R)-(2''-ethanal)azetidin-2-one (2.0 g) in tetrahydrofuran (40 ml). The reaction is allowed to proceed for 15 minutes at -78°C and is then worked up by adding acetic acid (2 ml), saturated sodium chloride

solution (60 ml) and extracting three times with 50 ml portions of ether. The ether extract is washed with brine, dried and evaporated. The residual oil is chromatographed on 70 g silica gel. Elution with 30:70 ethyl acetate-hexane affords the title compound as a colorless oil having IR:5.75 ; NMR: ($CDCl_3$) 5.94(dd,1H,J=8; 3 cps) 4.6(d,2H, J=6 cps), 5.3 (m,2H), 5.9 (m,1H); MS: 488, 490 (M+ -57).

F. <u>1-(t-Butyldimethyl)silyl-3(S)-[1'(R)-2,2,2-trichloroethoxycarbonyloxyethyl]-4(R)-(allyl-3''-keto-4''-butyrate)-azetidin-2-one:</u>

Chromium trioxide (12 g) is added in small portions to a solution of pyridine (19 g) in methylene chloride (300 ml). The resulting dark red solution is stirred vigorously for 15 minutes and is added to a solution of 1-(t-butyldimethyl)silyl-3(S)-[1'(R)-2,2,2-trichloroethoxycarbonyloxyethyl]-4(R)-(allyl-3''-hydroxy-4''-butyrate) azetidin-2-one (5.0 g) in methylene chloride (20 ml). The mixture is stirred until thin layer chromatography (silica-gel, 50:50 ether:hexane) indicates disappearance of starting material. The solution is filtered through 60 g silica gel and the eluate is washed with diluted hydrochloric acid, brine, dried and evaporated to afford the title compound as a colorless oil having NMR: ($CDCl_3$) 3.4(s,2H). MS: 486, 488 (M+ -57)

G. <u>3-(S)-[1'(R)-Hydroxyethyl]-4(R)-(allyl-3''-keto-4''-butyrate)azetidin-2-one:</u>

A solution of 1-(t-butyldimethyl)silyl-3(S)-[1'(R)-2,2,2-trichloroethoxycarbonyloxyethyl]-4(R)-(allyl-3''-keto-4''-butyrate)azetidin-2-one (2.7 g) in a mixture of tetrahydrofuran (27 ml), glacial acetic acid (16 ml) and water (6.0 ml) is cooled to -20°C (dry ice/carbon tetrachloride) and stirred with zinc dust (3.0 g) added in portions over 1 hour. The suspension is stirred for 3 hours, diluted with ethylacetate (50 ml) and filtered. The filtrate is washed with cold 10% aqueous sodium bicarbonate, brine, dried and evaporated. The resultant oil is dissolved in tetrafuran (40 ml) and stirred with 20 ml 401-S Fluoride resin (obtained by Neutralizing Rohm & Haas AMBERLITE 401-S resin with hydrofluoric acid and washing the resulting fluoride resin with water until washings are neutral) for 3 hours. The resin is then removed by filtration and the filtrate on evaporation affords the title compound having IR: 5.65, 5.78 ; NMR: ($CDCl_3$) 3.5(s, 2H).

E. 3(S)-[1'(R)-Hydroxyethyl]-4(S)-(allyl-2''-diaza-3''-keto-butyrate)azetidin-2-one:

A solution of 3(S)-[1'(R)-hydroxyethyl]-4(R)-(allyl-3''-keto-4''-butyrate)azetidin-2-one (0.68 g) and p-carboxybenzenesulfonylazide (0.6 g) in methylene chloride (10 ml) is cooled to 0°C and treated dropwise with triethylamine (0.9 ml). The mixture is then stirred at room temperature until thin layer chromatography (silica gel plate, 25% acetone/chloroform) indicated disappearance of starting material. The reaction mixture is then diluted with ether, washed with 10% aqueous sodium bicarbonate, brine, dried and evaporated to afford the title product as an oil having IR: 5.62, 5.75, 5.80

PREPARATION B

Bis (N-allyloxy carbonyl)-D-cystine bis-allyl ester

A solution of D-cystine (12 g) in a 4N sodium hydroxide (25 ml) at ice bath temperature is stirred while allyl chloroformate (10.6 ml) and 4N sodium hydroxide (25 ml) are added dropwise. The mixture is stirred for 30 mins. after the final addition and is then washed with 50 ml of ether. The aqueous phase is then acidified to pH 2 with 1N hydrochloric acid and extracted with 3 x 50 ml ether. The ether extracts are dried with sodium sulfate and evaporated. The residual colorless oil is dissolved in acetone (75 ml) containing triethylamine (9.6 ml) and stirred while allyl bromide (6.0 ml) is added dropwise. The mixture is stirred overnight, diluted with ethylacetate (75 ml) and brine (75 ml). The aqueous phase is extracted three times with ethyl acetate. The organic extracts are washed with 1N sodium hydroxide, 1N hydrochloric acid, water, dried with sodium sulfate and evaporated. The title product is crystallized from ether-hexane as colorless needles with a m.p. of 48-49°C and an $[\alpha]_D$ of +55° (chloroform).

(ii) N-allyloxycarbonyl-D-cysteine allyl ester

A suspension of zinc dust (8 g) in a solution of bis (N-allyloxylcarbonyl)-D-cystine bis allyl ester (7.56 g) in methanol (50 ml) at 0°C is stirred vigorously while adding concentrated hydrochloric acid (5 ml) in one portion. After 2 minutes the mixture is diluted with ice water and extracted with 3 x 50 ml. of chloroform. The extracts are dried over sodium sulfate and evaporated under reduced pressure to afford the title products.

EXAMPLE

(5R,6S,8R,2'S)-2-[2'-amino-2'-carboxyethyl)thio-6-(1-hydroxyethyl)-1-carbapen-2-em-3-carboxylic acid, sodium salt

A. A solution of 3(S)-[1'(R)-hydroxyethyl]-4(R)-(allyl-2''-diaza-3''-ketobutyrate)azetidin-2-one (0.5 g) in benzene (100 ml) containing rhodium acetate (0.004 g) is stirred at 80°C for 10 minutes until thin layer chromatography indicates complete reaction (silica-gel plates, 10:90 acetone/chloroform). The solution is cooled to room temperature, washed with water, brine, dried and evaporated to afford allyl(5R,6S)-[(8R)-1-hydroxyethyl]-1-carba-2-oxopenam-3-carboxylate (0.4 g) which is immediately dissolved in dichloromethane (5 ml) and cooled to 0°C. Then, to this is added dropwise, with

stirring, diisopropylethylamine (0.2 g), followed by diphenylchlorophosphate (0.42 g). The mixture is stirred at 0°C until thin layer chromatography indicates completion of reaction to form allyl (5R,6S)-[(8R)-1-hydroxyethyl] -1-carba-2-diphenylphosphatoxypen-2-em-3-carboxylate. The mixture is then treated with N-allyloxycarbonyl-D-cystein allyl ester (0.4 g) and diisopropylethylamine (0.2 g). Stirring is continued until thin layer chromatography (silica gel, 5:95 acetone: chloroform) shows that reaction is complete. The mixture is then washed with brine, concentrated to 5 ml and immediately used in the next step.

E. The solution from the above step is diluted with a 1M solution of pyridinium formate in methylene chloride (7.5 ml) and stirred with tetrakis(triphenylphosphine) palladium (0.2 g) and triphenylphosphine (0.2 g) for 20 minutes. The resulting precipitate is then collected and washed with 6 times with methylene chloride by centrifugation and suspended in a 0.5M solution of sodium 2-ethylhexanoate in ethyl acetate (2 ml)) for 1 hour. The precipitate is then collected after washing by centrifugation, three times with ethyl acetate and then with ether and dried *in vacuo* to afford the title product having IR: 5.68, 6.1 .

The following formulations are to exemplify some of the dosage forms in which the compound of this invention may be employed. In each, the active ingredient is designated by the term "Drug" which is meant to indicate the compound of the invention.

Injectable Suspension Formulation

| | mg/mg |
|---|---|
| Sterile drug | 250.0 |
| Benzyl Alcohol | 9.0 |
| Methylparaben | 1.8 |
| Propylparaben | 0.2 |
| Sodium Carboxymethylcellulose | 5.0 |
| Polyethylene Glycol 4000 | 10.0 |
| Povidone | 5.0 |
| Sodium Citrate | 15.0 |
| Disodium Edetate | 0.1 |
| Water for Injection | q.s |
| To make | 1.0 ml |

Dissolve parabens in a portion of the water for injection by heating it to 65-70°C. Cool to 25-35°C. Charge and dissolve benzyl alcohol, sodium citrate, disodium edetate, PEG 4000, povidone and sodium carboxymethylcellulose. Filter the solution and sterilize by autoclaving. Make a slurry of the sterile active and passit through the mill. Bring the suspension to the final volume/weight and fill into sterile containers.

Capsule Formulation

| Item No. | Ingredient | mg/capsule | mg/capsule |
|---|---|---|---|
| 1 | Drug | 250 | 500 |
| 2 | Lactose, USP | 106 | 123 |
| 3 | Corn Starch, Food Grade | 40 | 70 |
| 4 | Magnesium Stearate, USP | 4 | 7 |
| | | 400 mg | 700 mg |

Mix Item No.s. 1, 2 and 3 in a suitable mixer for 10-15 minutes. Add Item No. 4 and mix for 1-3 minutes. Fill the above mixture into suitable 2-piece hard gelatin capsules.

Tablet Formulation

| Item No. | Ingredient | mg/tablet | mg/tablet |
|---|---|---|---|
| 1 | Drug | 250 | 500 |
| 2 | Lactose, USP | 106 | 112 |
| 3 | Corn starch, Food Guide as 10% paste in water | 20 | 40 |
| 4 | Corn starch, Food Guide | 20 | 40 |
| 5 | Magnesium Stearate | 4 | 8 |
| | | 400.0 mg | 800.0 mg |

Mix Item Nos. 1 and 2 in a suitable mixer for 10-15 minutes. Granulate the mixture with Item No. 3. Pass the wet granulation through a coarse screen (1/4"). Dry the wet granules for 8-12 hours at 40-50°C. Using a suitable mill, pass the dried granules through a medium screen (No. 12 to No. 16). Add Item No. 4 and mix for 10-15 minutes. Add Item No. 5 and mix further for 1-3 minutes. Compress the mixture to appropriate size and weight on a suitable tablet machine.

CLAIMS

1. (5R,6S,8R,2'S)-2[(2'-amino-2'-carboxyethyl)thio]-6-(1-hydroxyethyl)-1-carbapen-2-em-3-carboxylic acid sodium salt.

2. A pharmaceutical composition comprising the compound of claim 1.

3. The use of the compound of claim 1, in the treatment of bacterial infection.

4. A method of preparing (5R,6S,8R,2'S) -2[(2'-amino-2'-carboxyethyl)thio]-6-(1-hydroxy ethyl)-1-carbapen-2-em-carboxylic acid sodium salt characterised in that

A: (5R,6S,8R,2'S) -2[2'-amino-2'-carboxyethyl)thio]-6-(1-hydroxyethyl)-1-carbapen-2-em-3-carboxylic acid is transformed into its sodium salt;

B: a compound of general formula I

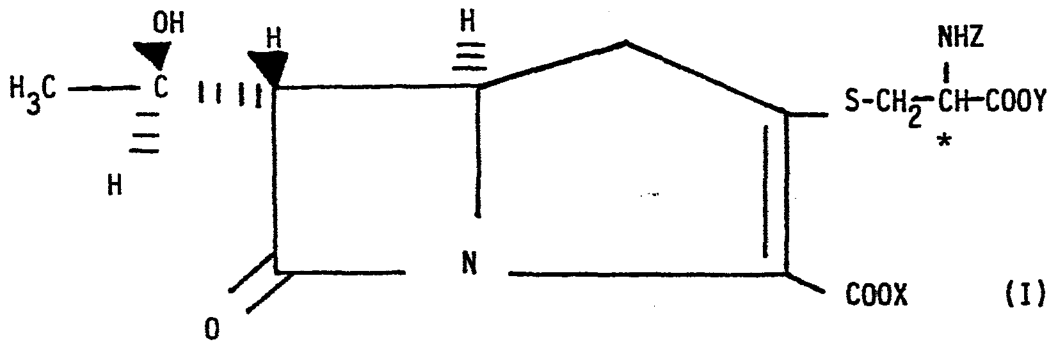

(I),

wherein * indicates a chiral centre of S configuration, Z is hydrogen or an amino protecting group and one of X and Y is a carboxy protecting group and the other is hydrogen or a carboxy protecting group, is subjected to deprotection by catalytic dehydrogenation in the presence of sodium cation; or

0126780

C: a compound of formula I as defined above in which Z is hydrogen, is subjected to catalytic deprotection in the presence of sodium cation.

5. A method of making a pharmaceutical composition which comprises mixing the compound (5R,6S,8R,2'S) -2-[(2'-amino-2'-carboxyethylthio]-6-[1-hydroxy-ethyl)-1-carbapen-2-em-3-carboxylic acid sodium salt with a pharmaceutically acceptable carrier or excipient.

European Patent Office

**0126780**

Application number

# EUROPEAN SEARCH REPORT

EP 83 10 3507

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | EP-A-0 037 080 (MERCK)<br>* Pages 88,96 (compound 38) * | 1-3 | C 07 D 487/04<br>A 61 K 31/40 //<br>C 07 D 205/08<br>(C 07 D 487/04<br>C 07 D 209/00<br>C 07 D 205/00 ) |
| X | EP-A-0 038 869 (MERCK)<br>* Claims 1,19, especially page 142 (third compound) * | 1-3 | |
| D,X | EP-A-0 017 992 (MERCK)<br>* Claims, especially claim 26 (pages 243,249, third compound) * | 1-3 | |
| Y | EP-A-0 074 599 (TAKEDA)<br>* Pages 120,121, example 67; claims 1-3,12,14 * | 1-3 | |
| Y | EP-A-0 008 888 (BEECHAM)<br>* Pages 124-126, example 53; claims * | 1-3 | TECHNICAL FIELDS SEARCHED (Int. Cl. 3) |
| | ----- | | C 07 D 487/00<br>A 61 K 31/00 |

The present search report has been drawn up for all claims

| Place of search | Date of completion of the search | Examiner |
|---|---|---|
| THE HAGUE | 24-11-1983 | CHOULY J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82